# EUROPÄISCHE PATENTSCHRIFT

(11) **EP 1 398 341 B1**
(45) Veröffentlichungstag und Bekanntmachung des Hinweises auf die Patenterteilung: **23.04.2008**
(21) Anmeldenummer: 03019574.7
(22) Anmeldetag: 03.09.2003
(51) Int. Cl.: C08G 83/00, C09K 11/00, C09K 11/06, H01L 51/30, H05B 33/08

(54) **Organische Verbindungen mit Kern-Schale-Struktur**
Organic compounds with core-shell structure
Composés organiques avec structure de coeur-enveloppe

(30) Priorität: 13.09.2002 DE 10242715; 12.02.2003 DE 10305945
(43) Veröffentlichungstag der Anmeldung: 17.03.2004
(73) Patentinhaber: H.C. Starck GmbH, 38642 Goslar (DE)
(72) Erfinder: Kirchmeyer, Stephan, Dr., 51375 Leverkusen (DE); Ponomarenko, Sergei, Dr., Moskau 107589 (RU)
(74) Vertreter: Clauswitz, Kai-Uwe Wolfram

(56) Entgegenhaltungen:
- WO-A-01/59030
- US-A- 6 025 462
- KIM Y H ET AL: "HYPERBRANCHED POLYPHENYLENES" MACROMOLECULES, AMERICAN CHEMICAL SOCIETY. EASTON, US, Bd. 25, Nr. 21, 12. Oktober 1992 (1992-10-12), Seiten 5561-5572, XP000315427 ISSN: 0024-9297
- KIEBOOMS R ET AL: "SYNTHESIS, ELECTRICAL, AND OPTICAL PROPERTIES OF CONJUGATED POLYMERS" HANDBOOK OF ADVANCED ELECTRONIC AND PHOTONIC MATERIALS AND DEVICES, XX, XX, Bd. 8, 2001, Seiten 1-102, XP001029240

## Beschreibung

Die Erfindung betrifft organische Verbindungen, die eine Kem-Schale-Struktur aufweisen, ein Verfahren zu deren Herstellung sowie ihre Verwendung als Halbleiter in elektronischen Bauelementen.

Das Feld molekularer Elektronik hat sich in den letzten 15 Jahren mit der Entdeckung organischer leitender und halbleitender Verbindungen rapide entwickelt. In dieser Zeit wurden eine Vielzahl von Verbindungen gefunden, die halbleitende oder elektrooptische Eigenschaften aufweisen. Es ist allgemeines Verständnis, dass die molekulare Elektronik nicht konventionelle Halbleiterbausteine auf der Basis von Silizium verdrängen wird. Stattdessen geht man davon aus, dass molekulare elektronische Bauelemente sich neue Anwendungsgebiete eröffnen werden, in denen die Eignung zur Beschichtung großer Flächen, strukturelle Flexibilität, Prozessierbarkeit bei niedrigen Temperaturen und niedrigen Kosten benötigt werden. Halbleitende organische Verbindungen werden derzeit für Anwendungsgebiete wie organische Feld-Effekt-Transistoren (OFET's), organische Lumineszenzdioden (OLED's), Sensoren und photovoltaische Elemente entwickelt. Durch einfache Strukturierung und Integration von OFET's in integrierte organische Halbleiterschaltungen werden preiswerte Lösungen für intelligente Karten (smart cards) oder Preisschilder möglich, die sich bislang mit Hilfe der Silzium-Technologie aufgrund des Preises und der mangelnden Flexibilität der Siliziumbausteine nicht realisieren lassen. Ebenfalls könnten OFET's als Schaltelemente in großflächigen flexiblen Matrixanzeigen verwendet werden. Eine Übersicht über organische Halbleiter, integrierte Halbleiterschaltungen und deren Anwendungen ist beispielweise in Electronics 2002, Band 15, S. 38 beschrieben.

Ein Feldeffekttransistor ist ein Dreielektroden-Element, in dem die Leitfähigkeit eines dünnen Leitungskanals zwischen zwei Elektroden (genannt "Source" und "Drain") mittels einer an der dritten, durch eine dünne Isolatorschicht von dem Leitungskanal getrennten, Elektrode (genannt "Gate") kontrolliert wird. Die wichtigsten charakteristischen Eigenschaften eines Feldeffekt-Transistors sind die Mobilität der Ladungsträger, die entscheidend die Schaltgeschwindigkeit des Transistors bestimmen und das Verhältnis zwischen den Strömen im geschalteten und ungeschalteten Zustand, das sogenannte "On/Off ratio".

In organischen Feldeffekt-Transistoren sind bislang zwei große Klassen von Verbindungen verwendet worden. Sämtliche Verbindungen weisen fortlaufende konjugierte Einheiten auf und werden je nach Molekulargewicht und Aufbau in konjugierte Polymere und konjugierten Oligomere unterteilt.

Oligomere weisen in der Regel eine einheitliche molekulare Struktur und ein Molekulargewicht unter 10000 Dalton auf. Polymere bestehen in der Regel aus Ketten einheitlicher Repetitionseinheiten mit einer Molekulargewichtsverteilung. Jedoch besteht ein fließender Übergang zwischen Oligomeren und Polymeren.

Häufig wird mit der Unterscheidung zwischen Oligomeren und Polymeren zum Ausdruck gebracht, dass in der Verarbeitung dieser Verbindungen eine grundsätzliche Unterscheidung besteht. Oligomere sind häufig verdampfbar und werden über Aufdampfverfahren auf Substrate aufgebracht. Als Polymere werden häufig unabhängig von ihrer molekularen Struktur Verbindungen bezeichnet, die nicht mehr verdampfbar sind und daher über andere Verfahren aufgebracht werden. Bei Polymeren werden in der Regel Verbindungen angestrebt, die in einem flüssigen Medium, beispielsweise organischen Lösungsmitteln, löslich sind und sich dann über entsprechende Auftragsverfahren aufbringen lassen. Ein sehr verbreitetes Auftragsverfahren ist z.B. das Aufschleuderverfahren ("Spin-Coating"). Eine besonders elegante Methode ist das Aufbringen von halbleitenden Verbindungen über das Ink-Jet-Verfahren. In diesem Verfahren wird eine Lösung der halbleitenden Verbindung in Form feinster Tröpfchen auf das Substrat aufgebracht und getrocknet. Dieses Verfahren erlaubt während des Aufbringens die Strukturierung durchzuführen. Eine Beschreibung dieses Aufbringverfahrens für halbleitende Verbindungen ist beispielsweise in Nature, Band 401, S. 685 beschrieben.

Allgemein wird den nasschemischen Verfahren ein größeres Potenzial zugeschrieben, auf einfache Art und Weise zu preisgünstigen organischen integrierten Halbleiterschaltungen zu kommen.

Eine wichtige Voraussetzung zur Herstellung hochwertiger organischer Halbleiterschaltungen sind Verbindungen extrem hoher Reinheit. In Halbleitern spielen Ordnungsphänomene eine große Rolle. Behinderung an einheitlicher Ausrichtung der Verbindungen und Ausprägungen von Korngrenzen führt zu einem dramatischen Abfall der Halbleitereigenschaften, so dass organische Halbleiterschaltungen, die unter Verwendung nicht extrem hochreiner Verbindungen gebaut wurden, in der Regel unbrauchbar sind. Verbleibende Verunreinigungen können beispielsweise Ladungen in die halbleitende Verbindung injizieren ("Dotierung") und damit das On/Off-Ratio verkleinern oder als Ladungsfallen dienen und damit die Mobilität drastisch herabsetzen. Weiterhin können Verunreinigung die Reaktion der halbleitenden Verbindungen mit Sauerstoff initiieren und oxidierend wirkende Verunreinigungen können die halbleitenden Verbindungen oxidieren und somit möglicher Lager-, Verarbeitungs- und Betriebszeiten verkürzen.

Die in der Regel notwendigen Reinheiten sind solche, die durch die bekannten polymerchemischen Verfahren wie Waschen, Umfällen und Extraktion in der Regel nicht erreichbar sind. Oligomere können dagegen als molekular einheitliche und häufig flüchtige Verbindungen relativ einfach durch Sublimation oder Chromatographie gereinigt werden.

Einige wichtige Vertreter halbleitender Polymere sind im Folgenden beschrieben. Für Polyfluorene und Fluoren-copolymere, beispielsweise Poly(9,9-dioctylfluoren-co-bithiophen) (I) 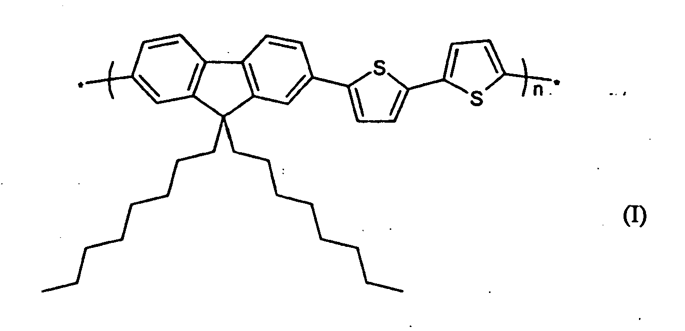 wurden Ladungsmobilitäten, im Folgenden auch kurz als Mobilitäten bezeichnet, bis 0,02 cm²/Vs erreicht (Science, 2000, Band 290, S. 2123), mit regioregulärem Poly(3-hexylthiophen-2,5-diyl) (II) 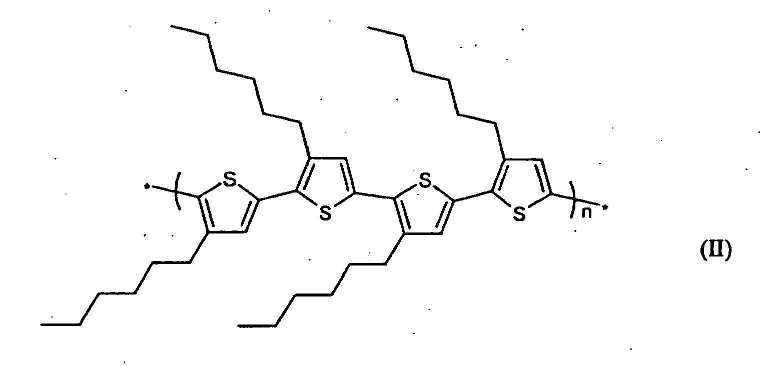 sogar Mobilitäten bis zu 0,1 cm²/Vs (Science, 1998, Band 280, S. 1741). Polyfluoren, Polyfluoren-Copolymere und Poly(3-hexylthiophen-2,5-diyl) bilden wie fast alle langkettigen Polymere nach Aufbringung aus Lösung gute Filme und sind daher einfach zu prozessieren. Als hochmolekulare Polymere mit einer Molekulargewichtsverteilung können sie jedoch nicht durch Vakuumsublimation und nur schwer durch Chromatographie gereinigt werden.

Wichtige Vertreter oligomerer halbleitender Verbindungen sind beispielsweise Oligothiophene, insbesondere solche mit endständigen Alkylsubstituenten gemäß Formel (III) 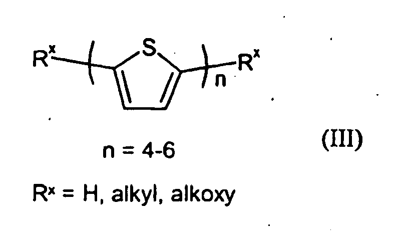 und Pentacen (IV)

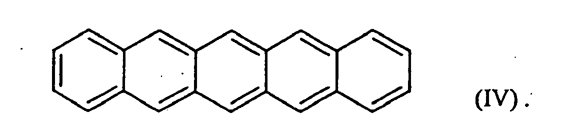

Typische Mobilitäten, für z.B. α,α'-Dihexylquarter-, -quinque- und -sexithiophen liegen bei 0.05 - 0.1 cm²/Vs.

Mesophasen, insbesondere flüssigkristalline Phasen scheinen in halbleitenden organischen Verbindungen eine besondere Rolle zu spielen, die aber bislang von den Fachleuten nicht vollständig verstanden wurde. Beispielsweise wird die bislang höchste Mobilität für Kristalle aus α,α'-Dihexylquarterthiophene berichtet (Chem. Mater., 1998, Band 10, S. 457), wobei diese Kristalle bei einer Temperatur von 80°C aus einer enantiotropen flüssigkristallinen Phase kristallisierten (Synth. Met., 1999, Band 101, S. 544). Besonders hohe Mobilitäten kann man bei Verwendung von einzelnen Kristallen erhalten, z.B. wurde eine Mobilität von 1,1 cm²/Vs für einzelne Kristalle von α,α'-Sexithiophene beschrieben (Science, 2000, Band 290, S. 963). Werden Oligomere aus Lösung aufgebracht, so sinken die Mobilitäten meist stark ab.

In der Regel wird das Absinken der halbleitenden Eigenschaften bei Prozessierung von oligomeren Verbindungen aus Lösung auf die mäßige Löslichkeit und geringe Tendenz zur Filmbildung der oligomeren Verbindungen zurückgeführt. So werden Inhomogenitäten beispielsweise auf Ausfällungen während der Trocknung aus der Lösung zurückgeführt (Chem. Mater., 1998, Band 10, S. 633).

Es hat daher Versuche gegeben, die guten Prozessierungs- und Filmbildungseigenschaften von halbleitenden Polymeren mit den Eigenschaften von halbleitenden Oligomeren zu verbinden. In US-A 6.025.462 werden leitfähige Polymere mit Sternstruktur beschrieben, die aus einem verzweigten Kem und einer Schale aus konjugierten Seitengruppen bestehen. Diese weisen allerdings einige Nachteile auf. Werden die Seitengruppen aus seitlich unsubstituierten konjugierten Strukturen gebildet, so sind die resultierenden Verbindungen schwer- oder unlöslich und nicht verarbeitbar. Substituiert man die konjugierten Einheiten mit Seitengruppen, so führt dies zwar zu einer verbesserten Löslichkeit, jedoch bewirken die Seitengruppen durch ihren sterischen Bedarf eine innere Unordnung und morphologische Störungen, welche die halbleitenden Eigenschaften dieser Verbindungen beeinträchtigen.

WO 02/26859 beschreibt Polymere aus einem konjugierten Rückrad, an das aromatische konjugierte Ketten angebracht werden. Die Polymere tragen Diarylamin Seitengruppen, die eine Elektronenleitung ermöglichen. Diese Verbindungen sind jedoch aufgrund der Diarylaminseitengruppen als Halbleiter ungeeignet.

Es besteht also weiterhin Bedarf an Verbindungen, welche die Eigenschaften von organischen halbleitenden Oligomeren und Polymeren verbinden.

Die Aufgabe bestand daher darin organische Verbindungen zur Verfügung zu stellen, die aus gängigen Lösungsmitteln prozessiert werden können und gute halbleitende Eigenschaften besitzen. Solche organischen halbleitenden Verbindungen würden sich hervorragend für die großflächige Beschichtung eignen.

Wünschenswert wäre, dass die Verbindungen hochwertige Schichten einheitlicher Dicke und Morphologie bilden und für elektronische Anwendungen geeignet sind.

Es wurde nun überraschend gefunden, dass organische Verbindungen die gewünschten Eigenschaften aufweisen, wenn sie eine Kern-Schale-Struktur aufweisen enthaltend einen Kern aufgebaut aus multifunktionalen Einheiten und eine Schale aus linearen konjugierten oligomeren Ketten, welche jeweils an der endständigen Verknüpfungsstelle durch eine flexible nichtkonjugierte Kette abgesättigt sind.

Gegenstand der Erfindung sind daher Verbindungen **dadurch gekennzeichnet, dass** sie eine Kern-Schale-Struktur aufweisen enthaltend einen Kern aufgebaut aus multifunktionalen Einheiten und eine Schale aus linearen konjugierten oligomeren Ketten, welche jeweils an der endständigen Verknüpfungsstelle durch eine flexible nichtkonjugierte Kette abgesättigt sind, wobei die Kern-Schale-Struktur eine solche der allgemeinen Formel (Z) ist,

K(̵L-R)ₙ (Z)

worin
- K: ein n-funktionaler Kern, ist.
- L: eine lineare konjugierte oligomere Kette enthaltend 2 bis 10 Einheiten der allgemeinen Formeln (VI-a), (VI-b) oder (VI-c) ist, 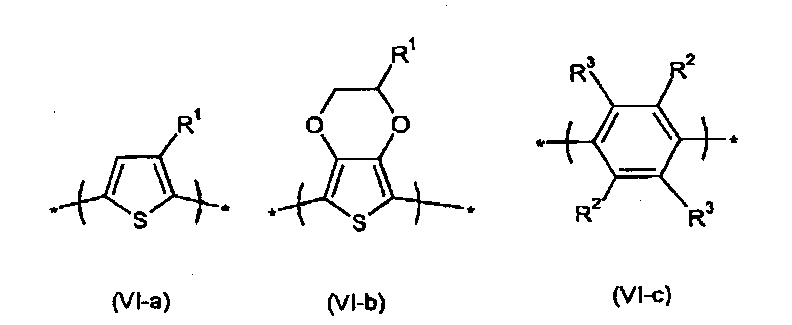 wobei
- R¹, R² und R³: jeweils für Wasserstoff stehen.
- R: für geradkettige oder verzweigte C₂-C₂₉-Alkylreste, einfach oder mehrfach ungesättigte C₂-C₂₀-Alkenylreste, C₂-C₂₀-Alkoxyreste, C₁-C₂₀-Aralkylreste oder C₂-C₂₀-Oligo-oder C₂-C₂₀-Polyetherreste steht,
- n: für eine ganze Zahl größer oder gleich 3, bevorzugt größer oder gleich 6 steht.

Dabei wird die Schale aus den n linearen konjugierten durch R abgesättigten Ketten L gebildet.

Für beispielsweise n gleich 3 oder 6 sind dies Strukturen der Formeln (Z-3) oder (Z-6) 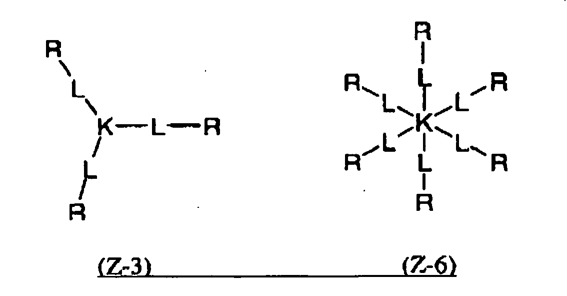 worin K, L und R oben genannte Bedeutung haben.

Die erfindungsgemäßen Verbindungen können in einer bevorzugten Ausführungsform Oligomere oder Polymere sein.

Im Rahmen der Erfindung handelt es sich bei der Kern-Schale-Struktur um eine solche auf molekularer Ebene, d.h. sie bezieht sich auf den Aufbau eines Moleküls.

Unter der endständigen Verknüpfungsstelle der linearen konjugierten oligomeren Kette ist die Stelle in der endständigen Einheit der linearen konjugierten oligomeren Kette zu verstehen, über die keine weilere Verknüpfung einer weiteren mehr erfolgt. Endständig ist im Sinne von am weitesten vom Kern entfernt zu verstehen.

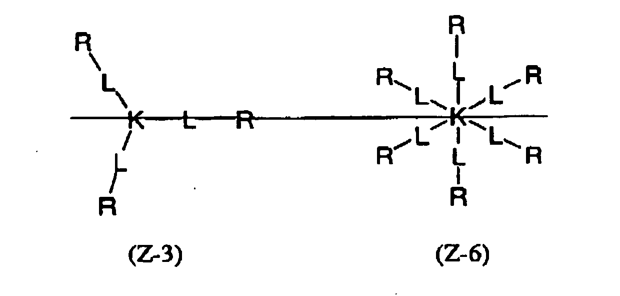

Derartige Verbindungen werden so konstruiert, dass ein aus multifunktionalen Einheiten aufgebauter, d.h. verzweigter Kern, lineare konjugierte oligomere Ketten und flexible nichtkonjugierte Ketten miteinander verbunden werden.

Der aus multifunktionalen Einheiten aufgebaute Kern weist dendritische oder hyperverzweigte Strukturen auf.

Hyperverzweigte Strukturen und ihre Herstellung sind dem Fachmann an sich bekannt. Hyperverzweigte Polymere oder Oligomere weisen eine besondere Struktur auf, die durch die Struktur der eingesetzten Monomeren vorgegeben ist. Als Monomere werden sog. ABn-Monomere eingesetzt, d.h. Monomere, die zwei verschiedene Funktionalitäten A und B tragen. Von diesen ist eine Funktionalität (A) pro Molekül nur einmal, die andere Funktionalität (B) mehrfach (n-mal) vorhanden. Die beiden Funktionalitäten A und B können miteinander unter Bildung einer chemischen Bindung umgesetzt, d.h polymerisiert werden. Aufgrund der Monomerstruktur entstehen bei der Polymerisation verzweigte Polymere mit einer baumartigen . Struktur, sogenannte hyperverzweigte Polymere. Hyperverzweigte Polymere weisen keine regelmäßigen Verzweigungsstellen, keine Ringe und praktisch ausschließlich B-Funktionalitäten an den Kettenenden auf. Hyperverzweigte Polymere, deren Struktur, die Frage der Verzweigung und deren Nomenklatur ist für das Beispiel von hyperverzweigten Polymeren auf der Basis von Siliconen in L.J. Mathias, T.W. Carothers, Adv. Dendritic Macromol. (1995), 2, 101-121 und den darin zitierten Arbeiten beschrieben.

Im Rahmen der Erfindung bevorzugte hyperverzweigte Strukturen sind hyperverzweigte Polymere.

Besonders bevorzugt weist der aus multifunktionalen Einheiten aufgebaute Kern jedoch dendritische Strukturen auf, da diese aufgrund ihres regelmäßigen Aufbaus besonders gut geeignet sind.

In Sinne der Erfindung sind dendritische Strukturen synthetische makromolekulare Strukturen, die man schrittweise durch Verknüpfung von jeweils 2 oder mehr Monomeren mit jedem bereits gebundenen Monomeren aufbaut, so dass mit jedem Schritt die Zahl der Monomer-Endgruppen exponentiell anwächst und am Ende eine kugelförmige Baumstruktur entsteht. Auf diese Weise entstehen dreidimensionale, makromolekulare Strukturen mit Gruppen, die Verzweigungspunkte aufweisen und sich von einem Zentrum in regelmäßiger Art und Weise bis zur Peripherie fortsetzen. Derartige Strukturen werden üblicherweise durch den Fachmann bekannte Verfahren Schicht für Schicht aufgebaut. Die Anzahl der Schichten wird üblicherweise Generation genannt. Die Anzahl der Verzweigungen in jeder Schicht wie auch die Anzahl endständiger Gruppen erhöhen sich mit steigender Generation. Dendritische Strukturen, Herstellmethoden und Nomenklatur sind dem Fachmann bekannt und beispielsweise in C.R.Newkome et.al., Dendrimers and Dendrons, Wiley-VCH, Weinheim, 2001 beschrieben.

Die im aus dendritischen Strukturen aufgebauten Kern, im Folgenden auch kurz als dendritischer Kern bezeichnet, einsetzbaren Strukturen sind prinzipiell diejenigen, die in US-A 6.025.462 beschrieben werden. Dies sind beispielsweise hyperverzweigte Strukturen wie Polyphenylene, Polyetherketone, Polyester, wie sie z.B. in US-A 5.183.862, US-A 5.225.522 und US-A 5.270.402 beschrieben werden, Aramide, wie sie z.B. in US-A 5.264.543 beschrieben werden, Polyamide, wie sie z.B. in US-A 5.346.984 beschrieben werden, Polycarbosilane oder Polycarbosiloxane, wie z.B. in US 6.384.172 beschrieben werden, oder Polyarylene, wie sie z.B. in US-A 5.070.183 oder US-A 5.145.930 beschrieben werden, oder dendritische Strukturen wie beispielsweise Polyarylene, Polyarylenether oder Polyamidoamine, wie sie z.B. in US-A 4.435.548 und US-A 4.507.466 beschrieben werden, sowie Polyethylenimine, wie sie z.B. in US-A 4.631.337 beschrieben werden.

Es können aber auch andere strukturelle Einheiten zum Aufbau des dendritischen Kerns verwendet werden. Die Rolle des dendritischen Kerns besteht vorwiegend darin, eine Reihe von Funktionalitäten zur Verfügung zu stellen und damit eine Matrix zu bilden, an denen die linearen konjugierten oligomeren Ketten angebracht und somit in einer Kern-Schale-Struktur angeordnet werden können. Die linearen konjugierten oligomeren Ketten werden durch Anbringung an die Matrix vorgeordnet und erhöhen so ihre Wirksamkeit.

Der dendritische Kern weist eine Reihe von Funktionalitäten - im Sinne von Verknüpfungsstellen - auf, die zur Anknüpfung der linearen konjugierten oligomeren Ketten geeignet sind. Erfindungsgemäß weist der dendritische Kern ebenso wie der aus hyperverzweigten Strukturen aufgebaute Kern mindestens 3, bevorzugt jedoch mindestens 6 Funktionalitäten auf.

Bevorzugte Strukturen im dendritischen Kern sind 1.,3,5-Phcnylen-Einheiten (Formel V-a) und Einheiten der Formeln (V-b) bis (V-e), wobei mehrere gleiche oder verschiedene Einheiten der Formeln (V-a) bis (V-e) miteinander verknüpft sind.

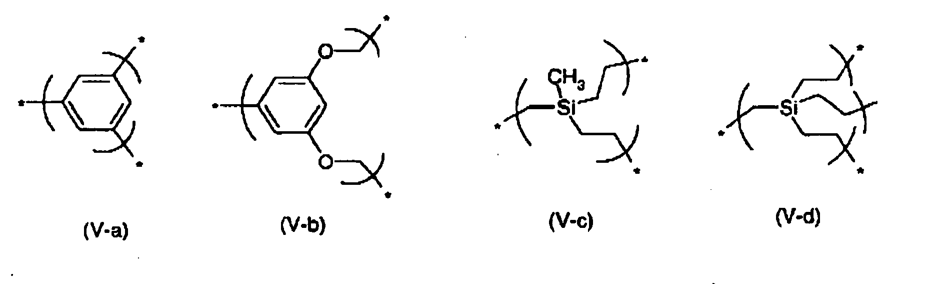

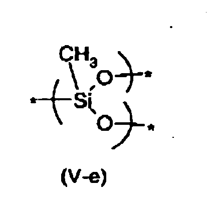

Die mit * gekennzeichneten Positionen in den Formeln (V-a) bis (V-e) sowie in weiteren im Folgenden verwendeten Formeln kennzeichnen die Verknüpfungsstellen. Die Einheiten (V-a) bis (V-e) werden über diese miteinander oder mit den linearen konjugierten oligomeren Ketten verknüpft.

Beispiele für dendritische Kerne aufgebaut aus Einheiten der Formel (V-a) sind folgende:

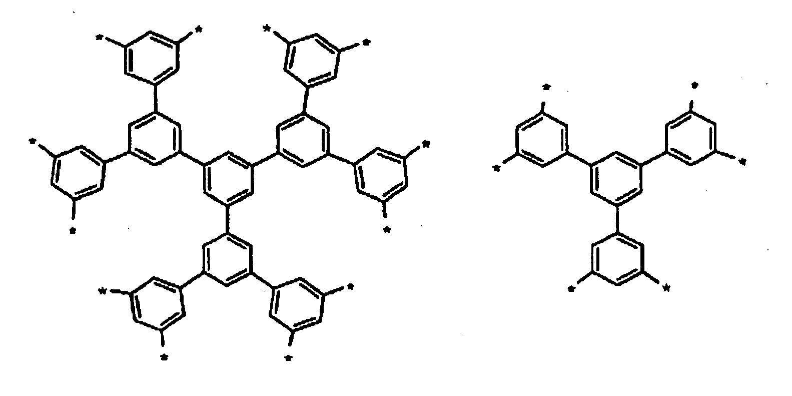

Über die mit * gekennzeichneten Positionen erfolgt die Verknüpfung mit den linearen konjugierten oligomeren Ketten.

Die Schale der erfindungsgemäßen Verbindungen wird aus linearen konjugierten oligomeren Ketten gebildet, welche an den Kern angeknüpft sind. Als lineare konjugierte oligomere Ketten sind prinzipiell sämtliche Ketten geeignet, die Strukturen aufweisen, die als leitfähige bzw. halbleitende Oligomere oder Polymere bekannt sind. Dies sind beispielsweise gegebenenfalls substituiert Polythiophene, Polyethylendioxythiophene oder, Polyphenylene, die als Homopolymere oder -oligomere oder als Copolymere oder oligomere eingesetzt werden können. Beispiele solcher Strukturen, die bevorzugt als lineare konjugierte oligomere Ketten eingesetzt werden können, sind Ketten aus 2 bis 10, besonders bevorzugt 2 bis 7 Einheiten der allgemeinen Formeln (VI-a) bis (VI-c), 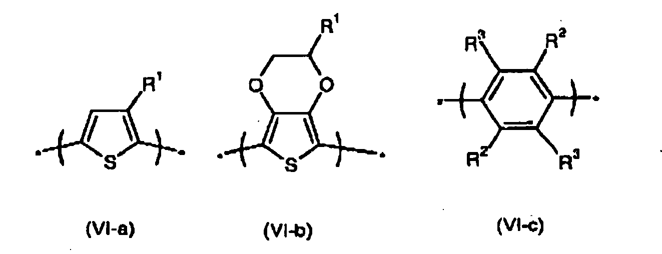 wobei
- R¹, R² und R³: jeweils für Wasserstoff stehen,

Die mit * gekennzeichneten Positionen der Formeln (VI-a) bis (VI-c) kennzeichnen die Verknüpfungsstellen, über die die Einheiten (VI-a) bis (VI-c) untereinander zur linearen konjugierten oligomeren Kette verknüpft sind bzw. an den jeweiligen Kettenenden an den Kern anknüpfen oder die flexible nichtkonjugierte Kette tragen.

Besonders bevorzugt sind lineare konjugierte oligomere Ketten mit Einheiten aus unsubstituierten 2,5-Thiophenen (VI-a) oder 2,5-(3,4-Ethylendioxythiophenen) (VT-b). Die vorangestellte Zahl 2.5 gibt an, über welche Positionen in den Einheiten die Verknüpfung erfolgt.

Beispielhaft sei hier die Verbindung der folgenden allgemeinen Formel (Z-6-1) genannt 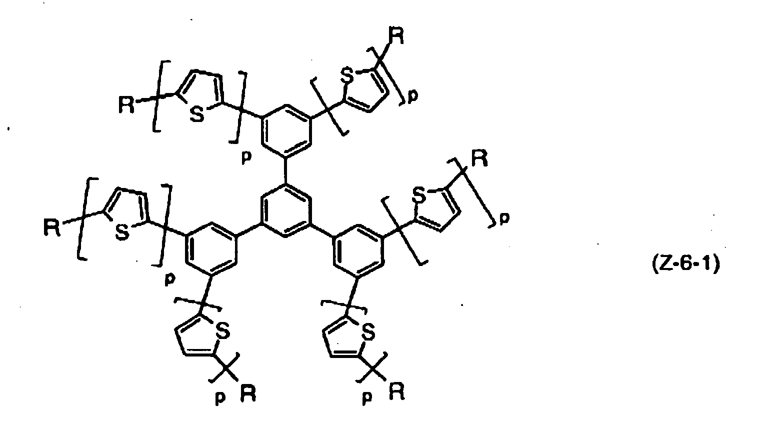 worin R die oben für die allgemeine Formel (Z) genannte Bedeutung hat und
- p: eine ganze Zahl von 2 bis 10, bevorzugt von 2 bis 7 ist.

Die linearen konjugierten oligomeren Ketten, in der allgemeinen Formel (Z) mit R bezeichnet, sind an der endständigen Verknüpfungsstelle jeweils durch eine flexible nichtkonjugierte Kette abgesättigt. Flexible Ketten sind solche, die eine hohe (intra)molekulare Beweglichkeit aufweisen, dadurch mit Lösemittelmolekülen gut wechselwirken und so eine verbesserte Löslichkeit erzeugen. Flexibel ist im Rahmen der Erfindung im Sinne von (intra)molekular beweglich zu verstehen. Die flexiblen nichtkonjugierten Ketten, welche die linearen konjugierten oligomeren Ketten an der endständigen Verknüpfungsstelle tragen, sind gegebenenfalls durch Sauerstoff unterbrochene geradkettige oder verzweigte aliphatische, ungesättigte oder araliphatische Ketten mit 2 bis 20 Kohlenstoffatomen, bevorzugt mit 6 bis 20 Kohlenstoffatomen. Bevorzugt sind aliphatische und oxyaliphatische Gruppen, d.h. Alkoxygruppen oder durch Sauerstoff unterbrochene geradkettige oder verzweigte aliphatische Gruppen wie Oligo- oder Polyether-Gruppen. Besonders bevorzugt sind unverzweigte C₂-C₂₀-Alkyl- oder C₂-C₂₀-Alkoxygruppen. Beispiele geeigneter Ketten sind Alkylgruppen wie n-Hexyl, n-Heptyl, n-Octyl, n-Nonyl, n-Decyl und n-Dodecylgruppen sowie Alkoxygruppen wie n-Hexyloxy-, n-Heptyloxy-, n-Octyloxy , n-Nonyloxy-, n-Decyloxy- und n-Dodecyloxygruppen.

Bevorzugte Ausführungsformen der vorliegenden Erfindung sind Verbindungen mit Strukturen, die im dendritischen Kern 1,3,5-Phenyleneinheiten, als lineare konjugierte oligomere Ketten unsubstituierte Oligothiophenketten und Oligo(3,4-ethylendioxythiophen)ketten mit 2 bis 4 Thiophen- bzw. 3,4-Ethylendioxythiophen-Einheiten sowie C₆-C₁₂-Alkylgruppen als flexible nichtkonjugierte Ketten enthalten.

Beispielhaft seien dazu die beiden folgenden Verbindungen der Formeln (XII) und (XIII) genannt:

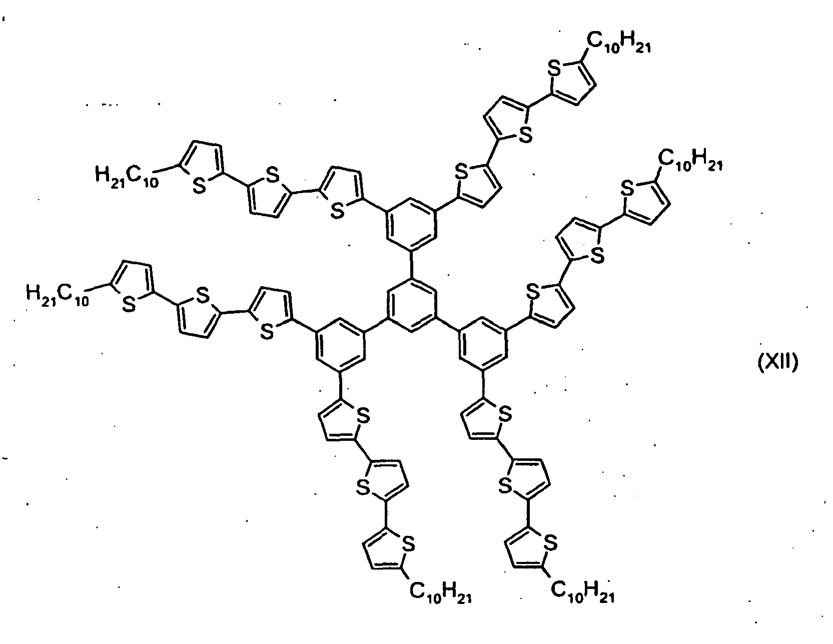

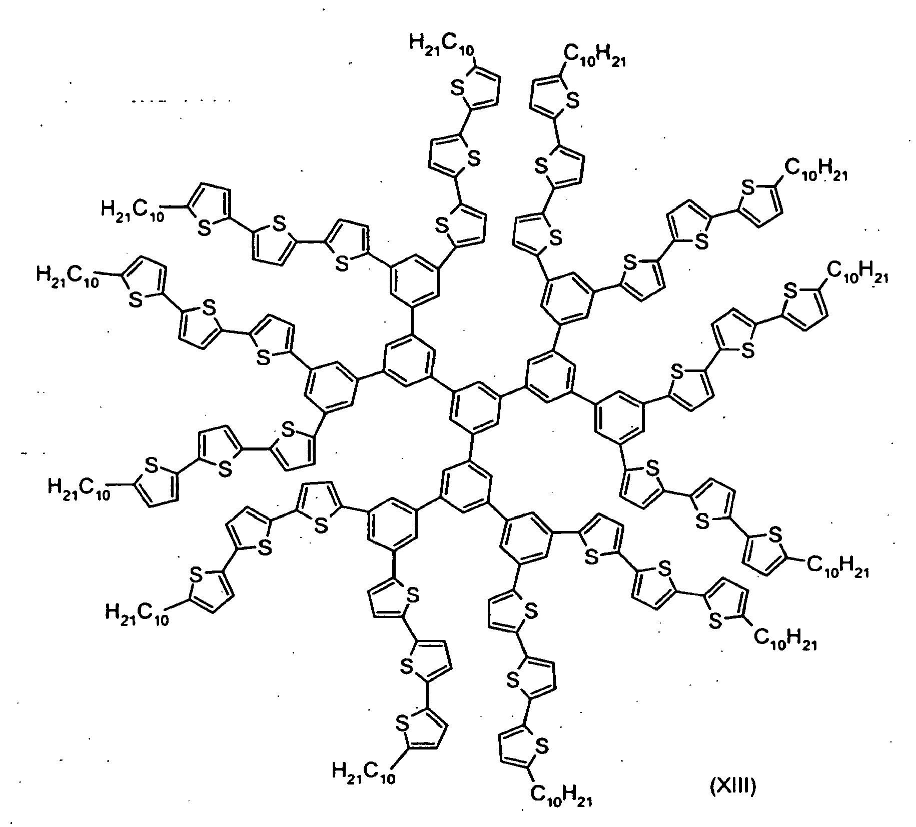

Die erfindungsgemäßen Verbindungen sind leitfähig oder halbleitend. Bevorzugt Gegenstand der vorliegenden Erfindung sind solche Verbindungen, die halbleitend sind. Besonders bevorzugt sind solche Verbindungen, die Mobilitäten von mindestens 10⁻⁴ cm²/Vs aufweisen

Die erfindungsgemäßen organischen Verbindungen sind in gängigen Lösungsmitteln, wie beispielsweise Chloroform, Toluol, Benzol, Diethylether, Dichlormethan oder Tetrahydrofuran, gut löslich und somit hervorragend für die Prozessierung aus Lösung geeignet. Überraschend ist insbesondere, dass auch erfindungsgemäße Verbindungen mit unsubstituierten Thiophen- bzw. 3,4-Ethylendioxythiophen-Einheiten in den linearen konjugierten oligomeren Ketten eine sehr gute Löslichkeit zeigen ohne das durch sterisch anspruchvolle Seitenketten die innere Ordnung bzw. Morphologie gestört wird. Entsprechend besitzen die erfindungsgemäßen organischen Verbindungen gute halbleitende sowie außerdem hervorragende Filmbildungseigenschaften. Sie eignen sich daher sehr gut für eine großflächige Beschichtung. Die erfindungsgemäßen organischen halbleitenden Verbindungen weisen weiterhin eine hervorragende thermischen Stabilität und gutes Alterungsverhalten auf.

Für die Herstellung der erfindungsgemäßen Verbindungen sind prinzipiell zwei unterschiedliche Verfahrenwege möglich, die als divergenter und konvergenter Herstellungsweg bezeichnet werden.

Im konvergenten Herstellungsweg wird in einem ersten Schritt die an einer Seite mit einer nichtkonjugierten flexiblen Kette abgesättigte lineare konjugierte oligomere Kette herstellt. Diese wird im Falle eines dendritischen Kerns an einen Monodendron genannten Baustein, d.h. einen Baustein, der einen Teil der dendritischen Struktur enthält und zu einer dendritischen Struktur zusammengefügt werden kann, geknüpft. Im letzten Herstellungsschritt werden mehrere Monodendronen zur Endstruktur vereinigt.

Im divergenten Herstellungsweg wird im ersten Schritt der dendritische Kern oder der hyperverzweigte Strukturen enthaltende Kern hergestellt. An diesen Kern können in einem anschließenden Schritt die jeweils mit einer nichtkonjugierten flexiblen Kette abgesättigten linearen konjugierten oligomeren Ketten angebracht werden.

Für Eigenschaften der erfindungsgemäßen Verbindungen ist der Herstellungsweg prinzipiell unerheblich. Innerhalb der beschriebenen Herstellungswege sind eine Reihe von Varianten möglich. So ist es beispielsweise möglich, die Reihenfolge der einzelnen Herstellungsschritte zu verändern und beispielsweise die Anknüpfung der nichtkonjugierten flexiblen Ketten an die linearen konjugierten oligomeren Ketten als letzten Herstellungsschritt durchzuführen.

Je nach herzustellender Struktur kann es beispielsweise jedoch sinnvoll sein, die Anknüpfung der nichtkonjugierten flexiblen Ketten an die linearen konjugierten oligomeren Ketten zu einem frühen Zeitpunkt durchzuführen, da die flexiblen Ketten die Löslichkeit der Bausteine erhöhen und dadurch die Herstellung der erfindungsgemäßen Verbindungen erleichtern.

Zum Aufbau des aus multifunktionalen Einheiten aufgebauten Kerns, zur Verknüpfung dessen mit den linearen konjugierten oligomeren Ketten und zur Anknüpfung der nichtkonjugierten flexiblen Ketten an diese stehen eine Reihe von chemischen Reaktionen zur Verfügung, die dem Fachmann prinzipiell bekannt sind. Vorzugsweise werden als chemische Reaktionen metallorganische Reaktionen ausgeführt. Diese besitzen den Vorteil, dass sie in der Regel unter milden Reaktionsbedingungen und hochselektiv verlaufen und hohe Reaktionsausbeuten erhalten werden.

Weiterhin Gegenstand der Erfindung ist somit ein Verfahren zur Herstellung der erfindungsgemäßen Verbindungen dadurch gekennzeichnet, dass diese durch metallorganische Reaktionen hergestellt werden.

Für metallorganische Reaktionen ist es notwendig, entsprechende Funktionalitäten in den dendritischen oder hyperverzweigten Kern, die linearen konjugierten oligomeren Ketten und die flexiblen nichtkonjugierten Ketten einzubringen und diese anschließend miteinander zu verknüpfen.

Derartige Funktionalitäten sind beispielsweise Halogengruppen, beispielsweise Chlor-, Brom- und Iodgruppen, vorzugsweise Bromgruppen, zinnorganische Gruppierungen wie z.B. die Trimethyl- oder Triethylzinngruppe, siliziumorganische Gruppierungen wie z.B. die Trimethylsilan- oder Triethylsilangruppe oder bororganische Gruppierungen wie z.B. Boronsäuren.

Besonders bevorzugte metallorganische Reaktionen zum Kuppeln der einzelnen Bauteile der erfindungsgemäßen Verbindungen sind die Kumada-Kupplung, in der zwei Bromgruppen über Grignard-Verbindungen unter Verwendung von PalladiumKatalysatoren, wie z.B. 1, 1 -Bis(diphenylposphino)ferrocendichlorpalladium(II), gekuppelt werden und die Suzuki-Kupplung, in der borhaltige Gruppen mit Bromgruppen basisch unter Verwendung von Palladiumkatalysatoren gekuppelt werden. Beide Kupplungsreaktionen sind dem Fachmann in ihrer Durchführung bekannt.

Bevorzugt werden die Zwischenstufen zwischen den einzelnen Herstellungsschritten sowie die Endverbindungen gereinigt. Dies kann nach den bekannten Methoden der Destillation, Sublimation, Rekristallisation, Extraktion, Umfällung, Waschen oder Chromatographie erfolgen. Bevorzugt werden Zwischenstufen und Endverbindungen durch Destillation, Sublimation und Chromatographie gereinigt, da hierdurch die höchsten Reinheiten erhalten werden können.

Dies bietet gegenüber den bekannten halbleitenden Polymeren den Vorteil, das über einfache gängige Reinigungsverfahren die erfindungsgemäßen Verbindungen in hohen Reinheiten hergestellt werden können und so für die Anwendung in der Halbleitertechnik geeignet sind.

Die erfindungsgemäßen Verbindungen können Mesophasen (mesomorphe Phasen), d.h. physikalische Phasen zwischen dem festen und flüssigen Zustand bilden. Diese werden auch als flüssigkristalline Phasen bezeichnet und unterstützen eine Vorordnung der erfindungsgemäßen Verbindungen. Bevorzugt bilden die erfindungsgemäßen Verbindungen flüssigkristalline Phasen im Bereich zwischen 50°C und 300°C, ganz besonders bevorzugt zwischen 80°C und 180°C.

Die erfindungsgemäßen Verbindungen sind in üblichen Lösungsmittel, z.B. in Chloroform, Toluol, Benzol, Diethylether, Dichlormethan oder Tetrahydrofuran, zu mindestens 0.1 % bevorzugt mindestens 1 %, besonderes bevorzugt mindestens 5 % löslich.

Die erfindungsgemäßen Verbindungen bilden aus Lösung hochwertige Schichten einheitlicher Dicke und Morphologie und sind daher für elektronische Anwendungen geeignet.

Weiterhin Gegenstand der Erfindung ist schließlich die Verwendung der erfindungsgemäßen Verbindungen als Halbleiter in elektronischen Bauelementen wie Feld-Effekt-Transistoren, lichtemittierenden Bauelementen, wie organischen Lumineszenzdioden, oder photovoltaischen Zellen, Lasern und Sensoren.

Bevorzugt werden die erfindungsgemäßen Verbindungen in Form von Schichten für diese Zwecke verwendet.

Um als Halbleiter sinnvoll eine Funktionalität sicherstellen zu können, weisen die erfindungsgemäßen Verbindungen eine ausreichende Mobilität, z.B. mindestens 10⁻⁴ cm ²/Vs auf. Zur Verwendung werden die erfindungsgemäßen Verbindungen auf geeignete Substrate, zum Beispiel auf mit elektrischen oder elektronischen Strukturen versehene Silizium-Wafer, Polymerfolien oder Glasscheiben, aufgetragen. Für den Auftrag kommen prinzipiell sämtliche Auftragsverfahren in Betracht. Vorzugsweise werden die erfindungsgemäßen Verbindungen aus flüssiger Phase, d.h. aus Lösung, aufgetragen und das Lösungsmittel anschließend verdampft. Das Aufbringen aus Lösung kann nach den bekannten Verfahren, beispielsweise durch Sprühen, Tauchen, Drucken und Rakeln erfolgen. Besonders bevorzugt ist das Aufbringen durch Spin-Coating und durch Ink-Jet-Druck.

Die Schichten der erfindungsgemäßen Verbindungen können nach dem Aufbringen weiter modifiziert werden, beispielsweise durch eine Temperaturbehandlung, z.B. unter Durchlaufen einer flüssigkristallinen Phase oder zur Strukturierung z.B. durch Laserablation.

Weiterhin Gegenstand der Erfindung sind elektronische Bauelemente enthaltend die erfindungsgemäßen Verbindungen als Halbleiter.

### Beispiele

5-Decyl-2,2':5',2"-terthiophene (Synthesis, 1993, S.1099; Chem. Mater., 1993, Band 5, S.430) und (3,5-Dibromophenyl)trimethylsilane (J. Organomet. Chem., 1983, Band 215, S.149) wurden nach den bekannten zitierten Prozeduren hergestellt.

### Beispiel 1: (Zwischenstufe aus linearer konjugierter oligomerer Kette und abschließender Kette).

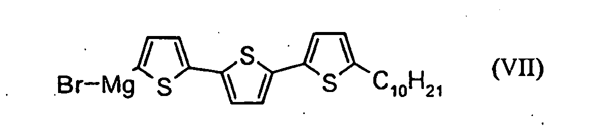

Herstellung von (5"-Decyl-2,2':5',2"-terthien-5-yl)-magnesiumbromid (VII): Zu einer Lösung aus 5-Decyl-2,2':5',2"-terthiophen (6.60 g, 14,4 mmol) in abs. (absolutiertem) THF (100 ml) wird unter N₂-Schutzgas unter Rühren bei 2°C über eine Spritze Butyllithium (1.6 M n-BuLi in Hexan, 8.8 ml, 14.0 mmol) zugetropft. Nach Beendigung der Zugabe lässt man die Lösung auf Raumtemperatur (23°C) erwärmen und rührt für eine Stunde nach. Anschließend wird erneut auf 2°C abgekühlt und MgBr₂Et₂0 (3.67 g, 14.2 mmol) in einem Guss zugegeben. Man lässt die Reaktionsmischung erneut auf Raumtemperatur erwärmen und rührt eine Stunde nach. (5"-Decyl-2,2':5',2"-terthien-5-yl)-magnesiumbromid wird nicht isoliert sondern die erhaltene Lösung wird direkt für die weiteren Umsetzungen verwendet.

### Beispiel 2: (Herstellung einer silanfuntionellen Monodendron-Zwischenstufe).

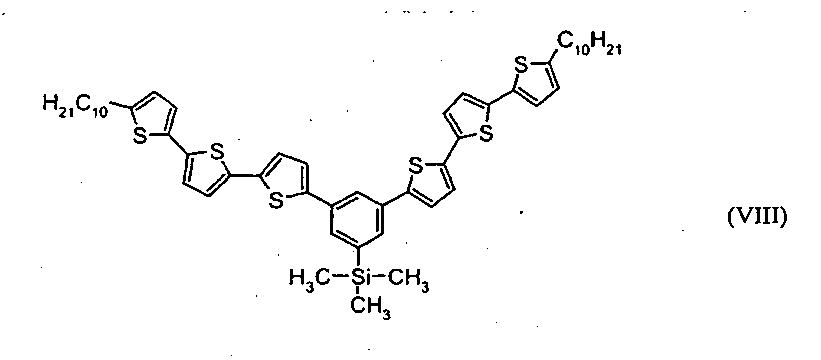

Herstellung von [3,5-Bis(5"-decyl-2,2':5',2"-terthien-5-yl)phenyl]-(trimethyl)silane (VIII): Eine frisch zubereitete Lösung aus (5"-Decyl-2,2':5',2"-terthien-5-yl)-magnesiumbromid (VII) (14.0 mmol) in abs. THF (Beispiel 1) wird bei Raumtemperatur tropfenweise zu einer Lösung aus (3,5-Dibromophenyl)trimethylsilan (1.54 g, 5 mmol) und Pd(dppf)Cl₂ (70 mg, 0.1 mmol) (dppf = diphenylphosphino) in 50 ml abs. THF gegeben. Nach Beendigung der Zugabe wird die Lösung für zwei Stunden gerührt. Die Vollständigkeit der Reaktion wird über DC kontrolliert. Anschließend wird die Reaktionsmischung in 300 ml kalten abs. Diethylether gegossen und 200 ml Eiswasser, enthaltend 20 ml 1 M (1 molare) HCl, werden zugegeben. Die Etherphase, welche einen gelben Niederschlag (das gewünschte Produkt) enthält, wird abgetrennt und mit drei Portionen zu je 100 ml Eiswasser nachgewaschen. Die Etherphase wird über einen G3 Glasfilter filtriert, das isolierte Produkt mit drei Portionen zu je 50 ml kaltem abs. Diethylether gewaschen und im Vakuum getrocknet. Man erhält 3.36 g Rohprodukt in Form dunkel gelber Kristalle. Nach Reinigung mittels Säulenchromatographie (Elutionsmittel Hexan-Chloroform 4:1) bei erhöhter Temperatur (40°C), und nachfolgender Rekristallisation aus Hexan, werden 2,64 g hellgelber Kristalle erhalten. Ausbeute: 57 %. Schmelzpunkt: 120 °C. ¹H NMR (400 MHz, CDCl_{3,} TMS/ppm): 0.353 (s, 9H), 0.884 (t, 6H, J=6.9), 1.20-1.45 (aberlappende Peaks, 28 H), 1.688 (m, 4H, J=7.3, M=5), 2.798 (t, 4H, J=7.6), 6.692 (d, 2H, J=3.9), 6.996 (d, 2H, J=3.9), 7.021 (d, 2H, J=3.9), 7.109 (d, 2H, 3.9), 7.156 (d, 2H, 3.4), 7.287 (d, 2H, 3.9), 7.623 (d, 2H, J=2.0), 7.763 (t, 1H, J=1.7).

### Beispiel 3: (Herstellung einer borfunktionellen Monodendron-Zwischenstufe)

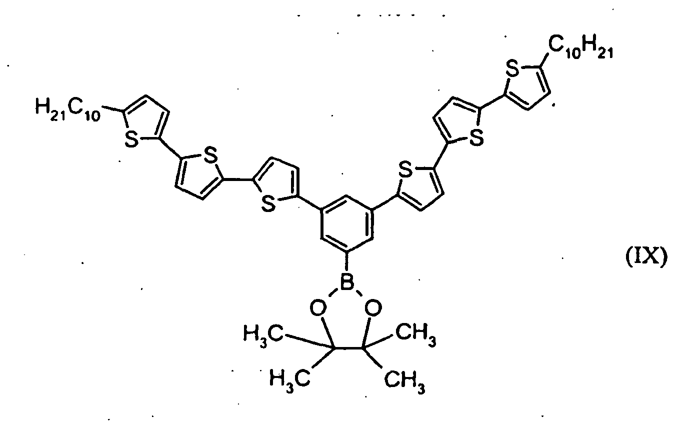

Herstellung von 2-[3,5-Bis(5"-decyl-2,2':5',2"-terthien-5-yl)phenyl]-4,4,5,5-tetramethyl-1,3,2-dioxaborolan (IX): Ein 100 ml Dreihalsrundkolben mit Rückflusskühler, Septum und Schutzgaseinlass wird mit N₂-Schutzgas gefüllt. Die Verbindung (VIII) (2,31 g, 2,5 mmol) und abs. Tetrachlormethan (40 ml) werden zugegeben. Anschließend werden 5 ml 1M BBr3-Lösung in Tetrachloromethan über eine Polypropylen-Einwegspritze in einer Portion über das Septum zugegeben und die Reaktionsmischung wird 40 h lang unter Rückfluss gekocht. Anschließend wird die Reaktionsmischung auf Raumtemperatur abgekühlt und mit einer Spritze in einen 500 ml Erlenmeyerkolben, enthaltend 250 ml 1 M NaOH-Lösung, überführt. Dann werden 200 ml Wasser zugegeben, die Mischung wird kräftig gerührt und die Wasserphase wird abdekantiert. Das verbleibende nasse Salz wird in einer Mischung aus Diethylether (200 ml), THF (100 ml) und 2 M HCl (300 ml) suspendiert und für 3 h kräftig gerührt bis sich zwei Phasen bilden. Die Etherphase wird abgetrennt und die wässrige Phase mit Diethylether nachgewaschen. Die vereinigten Etherphasen werden mit drei Portionen zu je 100 ml Wasser gewaschen und über Na₂SO₄ getrocknet. Das Lösungsmittel wird am Rotationsverdampfer entfernt. Es werden 4 g nasses Boronsäurederivat in Form eines braunen Feststoffes erhalten, wozu Pinakol (330 mg, 2,75 mmol) und abs. Toluol (100 ml) gegeben werden. Es wird über Nacht unter Rückfluss mit einem Dean-Stark Apparat (Wasserabscheider) gekocht. Nach Abkühlen und Entfernen des Toluols am Rotationsverdampfer werden 3 g Rohprodukt in Form eines braunen Feststoffes erhalten. Reinigung mittels Säulenchromatographie (Elutionsmittel: Toluol) ergibt 1,49 g sauberes Produkt als dunkel gelben Feststoff. Ausbeute: 61 %. ¹H NMR (400 MHz, CDCl_{3,} TMS/ppm): 0.884 (t, 6H, J=6.9), 1.20-1.45 (überlappende Peaks, 28 H), 1.388 (s, 12H), 1.688 (m, 4H, M=5, J=7.5), 2.798 (t, 4H, J=7.6), 6.692 (d, 2H, J=3.4), 6.995 (d, 2H, J=3.4), 7.071 (d, 2H, J=3.9), 7.104 (d, 2H, 3.9), 7.148 (d, 2H, 3.9), 7.334 (d, 2H, 3.4), 7.868 (t, 1H, J=1.7), 7.935 (d, 2H, J=2.0).

### Beispiel 4: (Herstellung einer silanfunktionellen Monodendron-Zwischenstufe)

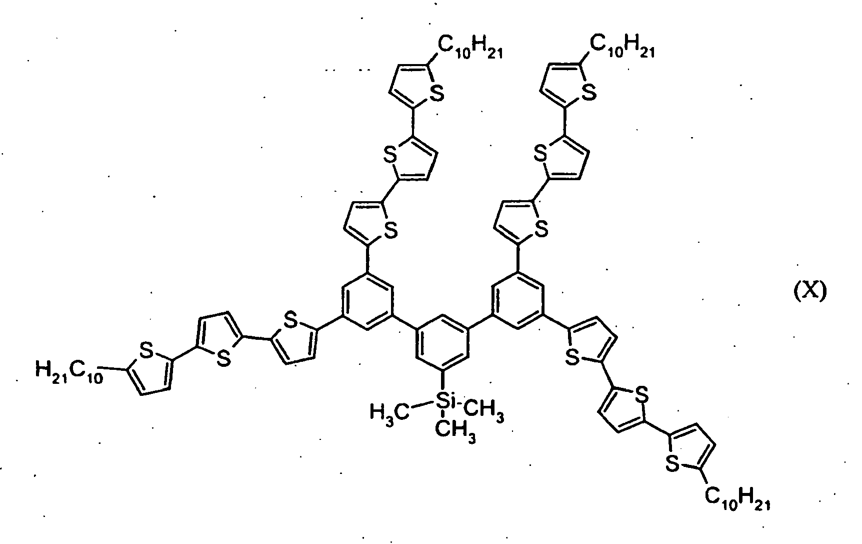

Herstellung von Trimethyl[3,3",5,5"-tetrakis(5"-decyl-2,2':5',2"-terthien-5-yl)-1,1':3',1"-terphenyl-5'-yl]silan (X): Ein 100-ml Dreihalskolben mit Magnetrührer, Rückflusskühler, Schutzgaseinlass und Septum wird unter N₂-Schutzgas mit (3,5-Dibromophenyl)trimethylsilan (215 mg, 0.7 mmol) befüllt und in einer Glovebox wird Pd(PPh₃)₄ (170 mg, 1.5 ×10⁻⁴ mol) zugegeben. Die Glasapparatur wird zusammengebaut und aus der Glovebox ausgeschleust. Daraufhin werden Lösungen der Verbindung (IX) (1.47 g, 1.5 mmol) in 30 ml Toluol und Na₂CO₃ (2 M aq. (wässrig), 10 ml) zubereitet und unter Durchleiten von N₂ desoxygeniert. Diese Lösungen werden in das Reaktionsgefäß gegeben und die Reaktionsmischung für 36 h unter N₂-Schutzgas zum Rückfluss erhitzt. Anschließend wird die Reaktionsmischung auf Raumtemperatur abgekühlt und in einen Kolben 100 ml Wasser und 300 ml CH₂Cl₂ gegossen. Die organische Phase wird abgetrennt und die wässrige Phase mit 100 ml CH₂Cl₂ nachgewaschen. Die vereinigten CH₂Cl₂-Phasen werden mit Wasser gewaschen, über MgSO₄ getrocknet und eingeengt. Das Produkt wird durch mehrfache Rekristallisation aus Hexan-Chloroform 4:1 Mischungen gereinigt und es werden 763 mg reines Produkt in Form eines braunen Feststoffes erhalten. Ausbeute: 59 %. Schmelzpunkt: 158 °C. GPC (Polystyrol-Standard): MP = 1540. ¹H NMR (400 MHz, CDCl₃, TMS/ppm): 0.421 (s, 9H), 0.882 (t, 12H, J=7.1), 1.20-1.45 (Überlappende Peaks, 56 H), 1.684 (m, 8H, M=5, J=7.6), 2.792 (t, 8H, J=7.6), 6.684 (d, 4H, J=3.9), 6.992 (d, 4H, J=3.4), 7.012 (d, 4H, J=3.9), 7.117 (d, 4H, 3.9), 7.179 (d, 4H, 3.4), 7.369 (d, 4H, 3.4), 7.753 (d, 4H, J=1.5), 7.802 (d, 2H, J=1.5), 7.816 (t, 2H, J=1.5), 7.865 (t, 1H, J=1.7).

### Beispiel 5: (Herstellung einer borfunktionellen Monodendron-Zwischenstufe)

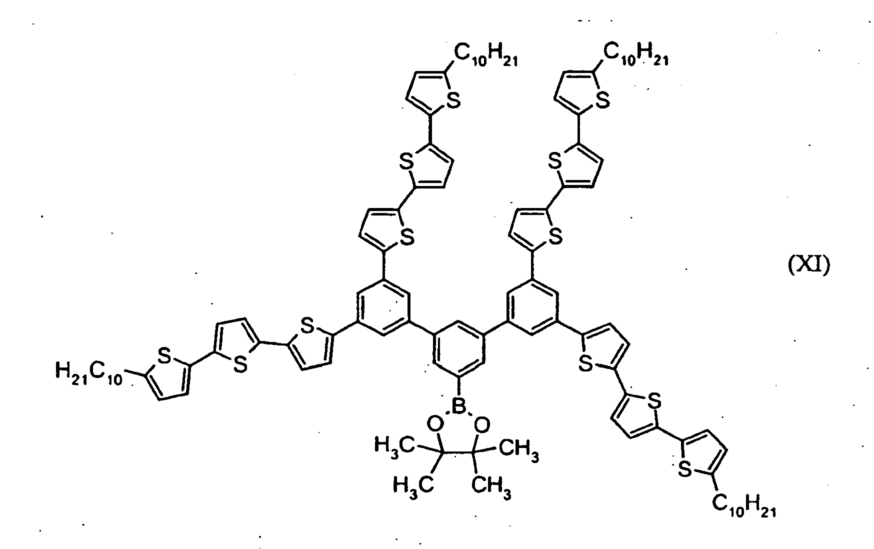

Herstellung von 4,4,5,5-Tetramethyl-2-[3,3",5,5"-tetrakis(5"-decyl-2,2':5',2"-terthien-5-yl)-1,1':3',1"-terphenyl-5'-yl]-1,3,2-dioxaborolan (XI): Die Verbindung wird nach einem vergleichbaren Prozess zu dem in Beispiel 3 beschriebenen aus der Verbindung (X) (610 mg, 0,33 mmol), 2 ml of 1M BBr₃-Lösung Tetrachloromethan und Pinakol (44 mg, 0,37 mmol) hergestellt. Waschvorgänge, wie in Beispiel 3 beschrieben, und Trocknen im Vakuum 650 mg Rohprodukt in Form eines braunen Feststoffes. Nach Reinigung mittels Säulenchromatographie (Elutionsmittel: Toluol) werden 310 mg sauberes Produkt als gelb-braune Kristalle erhalten. Ausbeute: 49 %. ¹H NMR (400 MHz, CDCl₃, TMS/ppm): 0.882 (t, 12H, J=7.1), 1.20-1.45 (überlappende Peaks, 56 H), 1.683 (m, 8H, M=5, J=7,6), 2.790 (t, 8H, J=7.6), 6.682 (d, 4H, J=3.4), 6.987 (d, 4H, J=3.4), 7.001 (d, 4H, J=3.9), 7.112 (d, 4H, 3.9), 7.175 (d, 4H, 3.9), 7,374 (d, 4H, 3.9), 7.785 (d, 4H, J=2.0), 7.799 (t, 2H, J=1.7), 7.988 (t, 1H, J=1.7), 8.123 (d, 2H, J=1.5).

### Beispiel 6 (Beispiel zur Herstellung einer erfindungsgemäßen Verbindung)

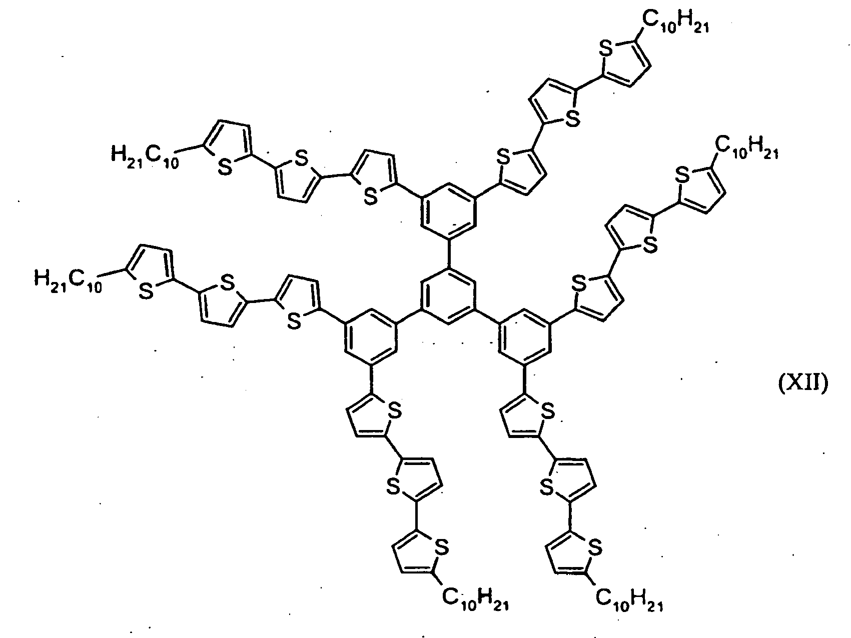

Herstellung von 1,3,5-Tris-[3,5-bis(5"-decyl-2,2':5',2"-terthien-5-yl)phenyl]benzol (XII): Die Verbindung wird nach einem vergleichbaren Prozess zu dem in Beispiel 4 beschriebenen aus der Verbindung (IX) (450 mg, 4.6 × 10⁻⁴ mol), 1,3,5-Tribromobenzol (40 mg, 1.27 × 10⁻⁴ mol) und Pd(PPh₃)₄ (17 mg, 1.5 × 10⁻⁵ mol) hergestellt. Nach Erhitzen zum Rückfluss für 16 h unter N₂-Schutzgas fällt gelber Niederschlag als Hauptprodukt aus. Die Reaktionsmischung wird auf Raumtemperatur gekühlt und in 100 ml Wasser und 400 ml CH₂Cl₂ gegossen. Die organische Phase, enthaltend den gelben Niederschlag, wird mit drei Portionen zu je 100 ml Wasser gewaschen, über einen G3 Glasfilter filtriert und der Filterrückstand (das Produkt) mit drei Portionen zu je 20 ml CH₂Cl₂ nachgewaschen. Nach Trocknen im Hochvakuum über Nacht werden 330 mg des sauberen Produktes erhalten. Ausbeute: 99 %. Schmelzpunkt: 202 °C.

### Beispiel 7: (Herstellung einer erfindungsgemäßen Verbindung)

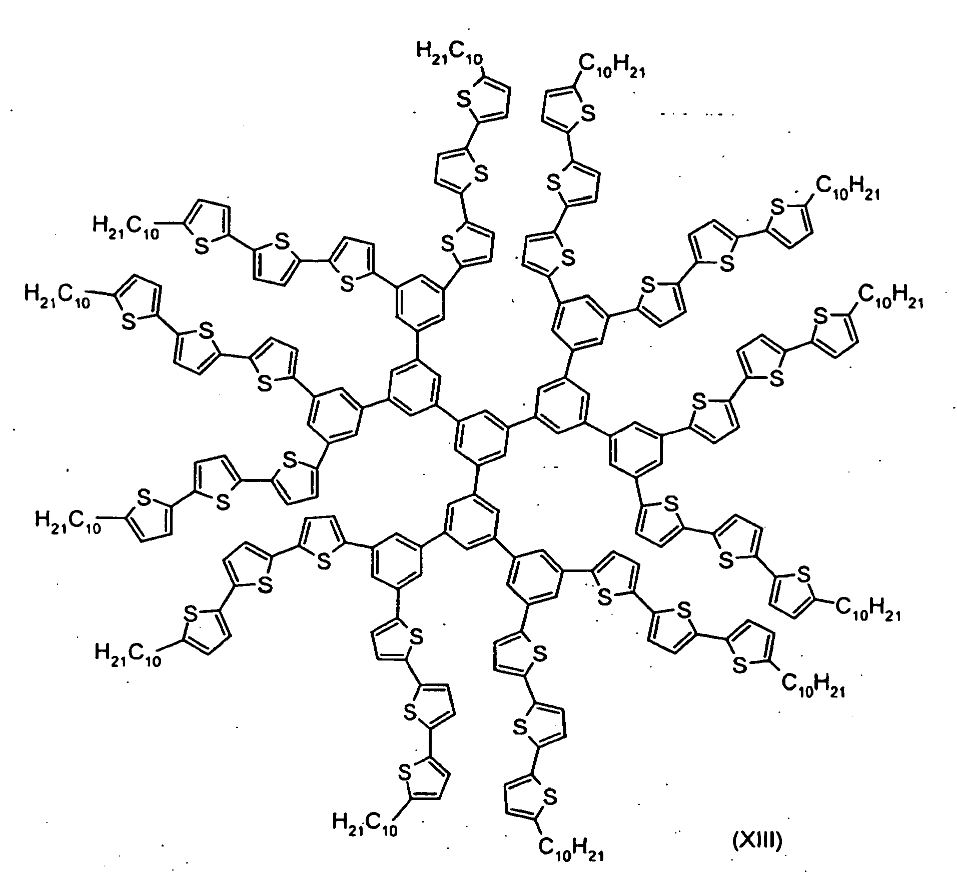

Herstellung von 1,3,5-Tris [3,3",5,5"-tetrakis(5"-decyl-2,2':5',2"-terthien-5-yl)-1,1':3',1"-terphenyl-5'-yl]benzol (XIII): Die Verbindung wird nach einem vergleichbaren Prozess zu dem in Beispiel 4 beschriebenen aus der Verbindung (XI) (250 mg, 1.3 × 10⁻⁴ mol), 1,3,5-Tribromobenzol (12 mg, 3.8 × 10⁻⁵ mol) und Pd(PPh₃)₄ (10 mg, 8.7 × 10⁻⁶ mol) hergestellt. Nach Erhitzen zum Rückfluss für 24 h unter N₂-Schutzgas wird die Verbindung gemäß dem Vorgang beschrieben in Beispiel 4 isoliert. 275 mg des Rohproduktes in Form eines braunen Feststoffes werden mittels Säulenchromatographie (Elutionsmittel: Toluol) gereinigt und 210 mg des sauberen Produktes als brauner glasartiger Feststoff erhalten. Ausbeute: 76 %. GPC (Polystyrol-Standard): MP = 6900, DPI = 1.02.

## Patentansprüche

1. Verbindungen **dadurch gekennzeichnet, dass** sie eine Kern-Schale-Struktur aufweisen enthaltend einen Kern aufgebaut aus multifunktionalen Einheiten und eine Schale aus linearen konjugierten oligomeren Ketten, welche jeweils an der endständigen Verknüpfungsstelle durch eine flexible nichtkonjugierte Kette abgesättigt sind, wobei die Kern-Schale-Struktur eine solche der allgemeinen Formel (Z) ist,
K(̵L-R)ₙ (Z)
worin
K ein n-funktionaler Kern ist,
L eine lineare konjugierte oligomere Kette enthaltend 2 bis 10 Einheiten der allgemeinen Formeln (VI-a), (VI-b) oder (VI-c) ist 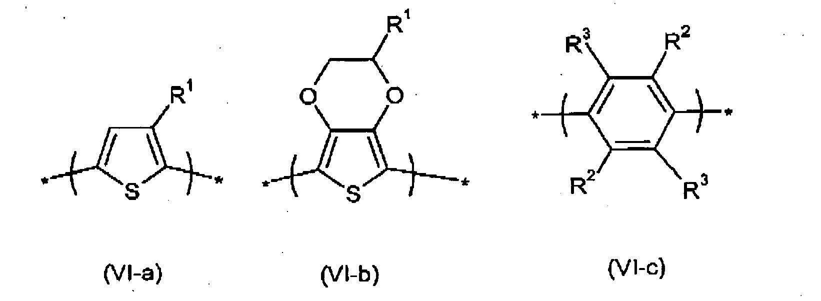 wobei
R¹,R² und R³ jeweils für Wasserstoff stehen,
R für geradkettige oder verzweigte C₂-C₂₀-Alkylrese, einfach oder mehrfach ungesättigte C₂-C₂₀-Alkenylreste, C₂-C₂₀-Alkoxyreste, C₂-C₂₀-Aralkylreste oder C₂-C₂₀-Oligo- oder C₂-C₂₀-Polyetherreste steht,
n für eine ganze Zahl größer oder gleich 3 steht.

2. Verbindungen gemäß Anspruch 1, **dadurch gekennzeichnet, dass** der Kern dendritische Strukturen enthält.

3. Verbindungen gemäß Anspruch 2, **dadurch gekennzeichnet, dass** der Kern als dendritische Strukturen 1,3,5-Phenylen-Einheiten enthält.

4. Verbindungen gemäß Anspruch 1, **dadurch gekennzeichnet, dass** der Kern hyperverzweigte Strukturen enthält

5. Verbindungen gemäß Anspruch 4, **dadurch gekennzeichnet, dass** der Kern als hyperverzweigte Struktur ein hyperverzweigtes Polymer enthält.

6. Verbindungen gemäß wenigstens einem der Ansprüche 1 bis 5, **dadurch gekennzeichnet, dass** die lineare konjugierte oligomere Ketten Einheiten der allgemeinen Formeln (VI-a) oder (VI-b) enthält.

7. Verbindungen gemäß wenigstens einem der Ansprüche 1 bis 6, **dadurch gekennzeichnet, dass** die linearen konjugierten oligomeren Ketten Ketten mit einer Kettenlänge von 2 bis 7 Einheiten sind.

8. Verbindungen gemäß wenigstens einem der Ansprüche 1 bis 7, **dadurch gekennzeichnet, dass** die linearen konjugierten oligomeren Ketten jeweils an den endständigen Verkaüpfungspositionen durch gleiche oder verschiedene, verzweigte oder unverzweigte Alkyl- oder Alkoxygruppen abgesättigt sind.

9. Verbindungen gemäß Anspruch 8, **dadurch gekennzeichnet, dass** die Alkyl- oder Alkoxygruppen unverzweigte C₂-C₂₀-Alkyl- oder C₂-C₂₀-Alkoxygruppen sind.

10. Verbindungen gemäß Anspruch 8 oder 9, **dadurch gekennzeichnet, dass** die Alkyl- oder Alkoxygruppen n-Hexyl, n-Decyl- oder n-Dodecylgruppen sind.

11. Verbindungen gemäß wenigstens einem der Ansprüche 1 bis 10, **dadurch gekennzeichnet, dass** sie bei Temperaturen zwischen 50°C und 300°C Mesophasen bilden.

12. Verbindungen gemäß wenigstens einem der Ansprüche 1 bis 11, **dadurch gekennzeichnet, dass** sie halbleitend sind.

13. Verbindungen gemäß wenigstens einem der Ansprüche 1 bis 12, **dadurch gekennzeichnet, dass** sie Mobilitäten von mindestens 10⁻⁴ cm²/Vs aufweisen.

14. Verfahren zur Herstellung der Verbindungen gemäß wenigstens einem der Ansprüche 1 bis 13, **dadurch gekennzeichnet, dass** sie durch metallorganische Reaktionen hergestellt werden.

15. Verfahren zur Herstellung der Verbindungen gemäß Anspruch 14, **dadurch gekennzeichnet, dass** sie unter Verwendung der Kumada-Kupplung hergestellt werden.

16. Verfahren zur Herstellung der Verbindungen gemäß Anspruch 14, **dadurch gekennzeichnet, dass** sie unter Verwendung der Suzuki-Kupplung hergestellt werden.

17. Verwendung der Verbindungen gemäß wenigstens einem der Ansprüche 1 bis 13 als Halbleiter in elektronischen Bauelementen.

18. Verwendung der Verbindungen gemäß Anspruch 17 als Halbleiter in Feld-Effekt-Transistoren, lichtemittierenden Bauelementen wie organischen Lumineszenzdioden, oder photovoltaischen Zellen, Lasern und Sensoren.

19. Verwendung der Verbindungen gemäß Anspruch 17 oder 18 in Form von Schichten, die aus Lösung aufgebracht werden.

20. Elektronische Bauelemente enthaltend Verbindungen gemäß wenigstens einem der Ansprüche 1 bis 13 als Halbleiter.

## Claims

1. Compounds **characterized in that** they have a core-shell structure comprising a core made up of multifunctional units and a shell of linear conjugated oligomeric chains which are each kept at the terminal linkage point by a flexible noncanjugated chain, where the core-shell structure is a structure of the formula (Z),
K(̵L-R)ₙ (Z)
where
K is an n-functional core,
L is a linear conjugated oligomeric chain containing from 2 to 10 units of the formulae (VI-a), (VI-b) or (VI-c) 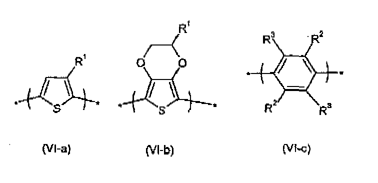 where
R¹, R² and R³ are each hydrogen,
R is a straight-chain or branched C₂-C₂₀-alkyl radical, a monounsaturated or polyunsaturated C₂-C₂₀₋alkenyl radical, a C₂-C₂₀-alkoxy radical, a C₂-C₂₀-aralkyl radical or a C₂-C₂₀-oligoether or C₂-C₂₀-polyether radical,
n is an integer greater than or equal to 3.

2. Compounds according to Claim 1, **characterized in that** the core comprises dendritic structures.

3. Compounds according to Claim 2, **characterized in that** the core contains 1,3,5-phenylene units as dendritic structures.

4. Compounds according to Claim 1, **characterized in that** the core comprises hyperbranched structures.

5. Compounds according to Claim 4, **characterized in that** the core contains a hyperbranched polymer as hyperbranched structure.

6. Compounds according to at least one of Claims 1 to 5, **characterized in that** the linear conjugated oligomeric chains contain units of the formulae (VI-a) or (VI-b).

7. Compounds according to at least one of Claims I to 6, **characterized in that** the linear conjugated oligomeric chains are chains having a chain length of from 2 to 7 units.

8. Compounds according to at least one of Claims 1 to 8, **characterized in that** the linear conjugated oligomeric chains are each capped at the terminal linkage positions by identical or different, branched or unbranched alkyl or alkoxy groups.

9. Compounds according to Claim 8, **characterized in that** the alkyl or alkoxy groups are unbranched C₂-C₂₀-alkyl or C₂-C₂₀-alkoxy groups.

10. Compounds according to Claim 8 or 9, **characterized in that** the alkyl or alkoxy groups are n-hexyl, n-decyl or n-dodecyl groups.

11. Compounds according to at least one of Claims 1 to 10, **characterized in that** they form mesophases at temperatures in the range from 50°C to 300°C.

12. Compounds according to at least one of Claims 1 to 11, **characterized in that** they are semiconductive.

13. Compounds according to at least one of Claims 1 to 12, **characterized in that** they have mobilities of at least 10⁻⁴ cm²/Vs.

14. Process for preparing compounds according to at least one of Claims I to 13, **characterized in that** they are prepared by organometallic reactions.

15. Process for preparing compounds according to Claim 14, **characterized in that** they are prepared using the Kumada coupling.

16. Process for preparing compounds according to Claim 14, **characterized in that** they are prepared using the Suzuki coupling.

17. Use of the compounds according to at least one of Claims 1 to 13 as semiconductors in electronic components.

18. Use of the compounds according to Claim 17 as semiconductors in field effect transistors, light-emitting components such as organic luminescence diodes, or photovoltaic cells, lasers and sensors.

19. Use of the compounds according to Claim 17 or 18 in the form of layers which are applied from solution.

20. Electronic components comprising compounds according to at least one of Claims 1 to 13 as semiconductors.

## Revendications

1. Composés, **caractérisés en ce qu'**ils présentent une structure à noyau-enveloppe comportant un noyau constitué d'unités multifonctionnelles et une enveloppe constituée de chaînes linéaires oligomères conjuguées qui sont saturées chacune à la position de liaison en bout de chaîne par une chaîne flexible non conjuguée, la structure à noyau-enveloppe étant une structure de formule générale (Z),
K(̵L-R)ₙ (Z)
dans laquelle
K est un noyau n-fonctionnel,
L est une chaîne linéaire oligomère conjuguée contenant 2 à 10 unités de formules générales (VI-a), (VI-b) ou (VI-c), 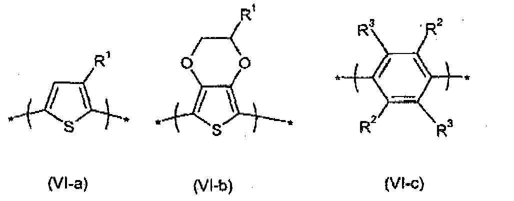 R¹, R² et R³ représentent chacun un atome d'hydrogène,
R représente des radicaux alkyle en C₂-C₂₀ à chaîne droite ou ramifiée, des radicaux alcényle en C₂-C₂₀ une ou plusieurs fois insaturés, des radicaux alcoxy en C₂-C₂₀, des radicaux aralkyle en C₂-C₂₀ ou des radicaux oligoéther en C₂-C₂₀ ou polyéther en C₂-C₂₀,
n représente un nombre entier égal ou supérieur à 3.

2. Composés selon la revendication 1, **caractérisés en ce que** le noyau contient des structures dendritiques.

3. Composés selon la revendication 2, **caractérisés en ce que** le noyau contient en tant que structures dendritiques des unités 1,3,5-phénylène.

4. Composés selon la revendication 1, **caractérisés en ce que** le noyau contient des structures hyper-ramifiées.

5. Composés selon la revendication 4, **caractérisés en ce que** le noyau contient en tant que structure hyper-ramifiée un polymère hyper-ramifié.

6. Composés selon au moins l'une des revendications 1 à 5, **caractérisés en ce que** les chaînes linéaires oligomères conjuguées contiennent des unités de formule générale (VI-a) ou (VI-b).

7. Composés selon au moins l'une des revendications 1 à 6, **caractérisés en ce que** les chaînes linéaires oligomères conjuguées sont des chaînes ayant une longueur de chaîne de 2 à 7 unités.

8. Composés selon au moins l'une des revendications 1 à 7, **caractérisés en ce que** les chaînes linéaires oligomères conjuguées sont saturées chacune en les positions de liaison en bout de chaîne par des groupes alkyle ou alcoxy ramifiés ou non ramifiés, identiques ou différents.

9. Composés selon la revendication 8, **caractérisés en ce que** les groupes alkyle ou alcoxy sont des groupes alkyle en C₂-C₂₀ ou alcoxy en C₂-C₂₀ non ramifiés.

10. Composés selon la revendication 8 ou 9, **caractérisés en ce que** les groupes alkyle ou alcoxy sont des groupes n-hPxyle, n-décyle ou n-dodécyle.

11. Composés selon au moins l'une des revendications 1 à 10, **caractérisés en ce qu'**ils forment des mésophases à des températures comprises entre 50°C et 300 °C.

12. Composés selon au moins l'une des revendications 1 à 11, **caractérisés en ce qu'**ils sont semi-conducteurs.

13. Composés selon au moins l'une des revendications 1 à 12, **caractérisés en ce qu'**ils présentent des mobilités d'au moins 10⁻⁴ cm²/Vs.

14. Procédé pour la préparation des composés selon au moins l'une des revendications 1 à 13, **caractérisé en ce qu'**on les prépare par des réactions organométalliques.

15. Procédé pour la préparation des composés selon la revendication 14, **caractérisé en ce qu'**on les prépare en utilisant le couplage de Kumada.

16. Procédé pour la préparation des composés selon la revendication 14, **caractérisé en ce qu'**on les prépare en utilisant le couplage de Suzuki.

17. Utilisation des composés selon au moins l'une des revendications 1 à 13, en tant que semi-conducteurs dans des composants électroniques.

18. Utilisation des composés selon la revendication 17, en tant que semi-conducteurs dans des transistors à effet de champ, des composants photoémetteurs, tels que des diodes luminescentes ou des cellules photovoltaïques, des lasers et des capteurs.

19. Utilisation des composés selon la revendication 17 ou 18, sous forme de couches qui sont appliquées à partir d'une solution.

20. Composants électroniques contenant des composés selon au moins l'une des revendications 1 à 13, en tant que semi-conducteurs.
